# EUROPÄISCHE PATENTSCHRIFT

(11) **EP 4 446 934 B1**
(45) Veröffentlichungstag und Bekanntmachung des Hinweises auf die Patenterteilung: **11.02.2026**
(21) Anmeldenummer: 24169776.2
(22) Anmeldetag: 11.04.2024
(51) Int. Cl.: G06K 7/00

(54) **LESEVORRICHTUNG ZUM ERFASSEN MEHRERER COMPUTERLESBARER CODES, LESESYSTEM UND VERFAHREN ZUM ERFASSEN MEHRERER COMPUTERLESBARER CODES**
READING DEVICE FOR READING SEVERAL COMPUTER-READABLE CODES, READING SYSTEM AND METHOD FOR READING SEVERAL COMPUTER-READABLE CODES
DISPOSITIF DE LECTURE POUR LA DÉTECTION DE PLUSIEURS CODES LISIBLES PAR ORDINATEUR, SYSTÈME DE LECTURE ET PROCÉDÉ DE DÉTECTION DE PLUSIEURS CODES LISIBLES PAR ORDINATEUR

(30) Priorität: 12.04.2023 DE 102023109249
(43) Veröffentlichungstag der Anmeldung: 16.10.2024
(73) Patentinhaber: Aesculap AG, 78532 Tuttlingen (DE)
(72) Erfinder: Koch, Jan, 78532 Tuttlingen (DE)
(74) Vertreter: Tergau & Walkenhorst Intellectual Property GmbH

(56) Entgegenhaltungen:
- WO-A1-2008/037094
- CN-A- 106 408 049
- DE-A1- 102021 120 340

## Beschreibung

Die Erfindung betrifft eine Lesevorrichtung gemäß Anspruch 1. Sie betrifft weiterhin ein Lesesystem gemäß Anspruch 12 und ein Verfahren gemäß Anspruch 13.

Die Nachverfolgung und Dokumentation von Medizinprodukten, die im Zusammenhang mit einem Patienten oder einem medizinischen Verfahren an einem Patienten verwendet werden, ist unerlässlich, um die Geräte - wobei es sich bei einem Gerät auch um jede Art von Medizinprodukt handeln kann - im Falle einer Infektion oder eines Geräterückrufs zurückverfolgen zu können. Es muss festgestellt werden können, welche spezifischen Artikel (Medizinprodukte), einschließlich ihrer Chargen- oder Seriennummer, im Zusammenhang mit einem Patienten verwendet, z. B. implantiert oder benutzt wurden. Eine Losnummer kann manchmal auch als Chargennummer bezeichnet werden.

Derzeit ist eine manuelle Dokumentation üblich, bei der abnehmbare Aufkleber von den Geräten entfernt werden. Häufig werden diese Aufkleber vom Personal auf die eine oder andere Weise eingesammelt. Es ist beispielsweise bekannt, dass die Aufkleber an den Kitteln befestigt werden, die während des Verfahrens getragen werden. Später, an einem Arbeitsplatz, der der Dokumentation dient, werden die Aufkleber entfernt und auf Formulare oder Papier geklebt, die dann wiederum archiviert und/oder digitalisiert werden können.

Für Medizinprodukte haben sich zwei Hauptstandards durchgesetzt, GS1 (Global Standards 1), z. B. GTIN (Global Trade Item Number), und HIBC (Health Industry Barcode). Für bestimmte Geräte wird auch die Pharmazentralnummer (PZN) verwendet. Um den richtigen Artikel dem gescannten Barcode zuordnen zu können, muss eine Datenbank mit Stammdaten aller möglicherweise verwendeten Artikel geführt werden.

Aus der WO 2022/112606 A1 sind ein Verfahren zum Erfassen mehrerer computerlesbarer Codes, vorzugsweise Barcodes und/oder 2D-Codes, eines medizinischen Produkts oder einer Verpackung eines medizinischen Produkts zur Unterstützung eines Benutzers sowie ein entsprechendes Lesegerät und Lesesystem bekannt.

Die DE 10 2021 120 340 A1 beschreibt einen Smart Port zum Erfassen und Auslesen von medizinischen Produkten mittels einer Vielzahl an modularen Modulen, welche jeweils mit unterschiedlichen Erfassungstechnologien vorgesehen und ausgebildet sind, sowie ein System mit einem Smart Port, einem Siebkorb und darin einbringbaren Instrumentenhaltern, welcher die medizinischen Produkte halten.

Aus der WO 2008 / 037 094 A1 ist ein System zur Übermittlung von Produktinformation für ein Produkt bekannt. Das System enthält ein Produkt mit einer elektronischen Controller zum Erfassen und/oder Speicherung von Produktinformationen, wobei die Produktinformation Standardinformationen über das Produkt und aktuelle Produktinformationen umfasst. Das System umfasst weiterhin eine Barcode-Erzeugungskomponente zur Erzeugung eines Barcodes zur Darstellung der Produktinformationen und eine Barcode-Aktualisierungseinrichtung zum Aktualisieren des Barcodes auf der Basis der Standardinformationen
über das Produkt und aktueller Produktinformationen.

Die CN 106 408 049 A beschreibt ein Verfahren zum Lesen und Erzeugen eines Produktcodes. Das Verfahren umfasst das Lesen einer ersten Kodierung aus dem ersten Ausführungskörper und das Erhalten einer ersten Ausführungsdatei aus der ersten Kodierung, wobei die Ausführungsinformationen die Ausführungsinformationen des ersten Ausführungskörpers, die Identitätsinformationen enthalten,

und das Erzeugen einer zweiten Kodierung eines zweiten Ausführungskörpers einschließlich einer zweiten Ausführungsdatei.

Nachteilig bei bekannten Systemen zur Erfassung von Codes von medizinischen Produkten ist, dass eine Übermittlung in vorgegebene Datenbanksysteme, insbesondere Krankeninformationssysteme (KIS) nicht möglich ist, da diese nur vorgegebene Datenformate akzeptieren. Es ist bekannt, Barcodescanner zu verwenden, diese können aber immer nur einen Code gleichzeitig senden und sind dazu ausgebildet, Daten nur an ein einziges System zu senden.

Der Erfindung liegt daher die Aufgabe zugrunde, eine Lesevorrichtung mit verbesserter Datenübertragung bereitzustellen. Weiterhin sollen ein entsprechendes Lesesystem und ein entsprechendes Verfahren angegeben werden.

In Bezug auf die Lesevorrichtung wird die oben genannte Aufgabe erfindungsgemäß gelöst durch eine Lesevorrichtung mit den Merkmalen von Anspruch 1. Dabei ist eine Lesevorrichtung zum Erfassen wenigstens eines computerlesbaren Codes eines medizinischen Produkts oder einer Verpackung eines medizinischen Produkts zur Unterstützung eines Benutzers vorgesehen, wobei die Lesevorrichtung eine Erfassungsvorrichtung umfasst, welche derart konfiguriert ist, dass sie ein Bild des medizinischen Produkts oder seiner Verpackung mit dem wenigstens einen Code erfasst und die in dem Code enthaltenen Produkt-Daten extrahiert, und wobei die Lesevorrichtung eine Interpretationsvorrichtung umfasst, welche konfiguriert ist, diese Produkt-Daten zu interpretieren und zu prüfen, ob vorgegebene Produkt-Informationen in den Produkt-Daten vorhanden sind, und wobei die Lesevorrichtung eine Codegenerierungsvorrichtung umfasst, welche konfiguriert ist, aus den Produkt-Daten einen Resultatcode zu erzeugen.

Vorteilhafte Ausgestaltungen der Erfindung sind Gegenstand der Unteransprüche.

Die Erfindung geht von der Überlegung aus, dass es bei der Erfassung von Codes von medizinische Produkten nützlich bzw. erforderlich ist, die entsprechenden Daten an externe Systeme, insbesondere das jeweilige Krankenhausinformationssystem (KIS), zu übermitteln. Das KIS weist allerdings im Regelfall eine spezifische und ggf. proprietäre Schnittstelle auf, sodass eine Übermittlung der erfassten Daten nicht ohne weiteres von einer Lesevorrichtung an das KIS möglich ist.

Wie nunmehr erkannt wurde, können diese Anforderungen erfüllt werden, indem die Lesevorrichtung eine Codegenerierungsvorrichtung umfasst, welche aus dem oder der erfasste Codes(s), einen gemeinsamen Code erzeugt, welcher dann an das KIS übermittelt werden kann. In dieser Weise wird ein gemeinsamer vom KIS verarbeitbarer Code erzeugt aus Daten, die aus einem oder mehreren Codes, welche unterschiedliche Formate haben können. Die Lesevorrichtung wird dadurch ertüchtigt, diesen Code an das externe System zu übertragen.

Erfindungsgemäß ist die Codegenerierungsvorrichtung konfiguriert, aus allen interpretierten Produkt-Daten einen gemeinsamen Resultatcode zu erzeugen. Dadurch kann sichergestellt werden, dass alle erfassten Produkt-Informationen in einem gemeinsamen Code zusammengefasst werden.

Unter dem Erfassen oder Scannen des Bildes ist bevorzugt zu verstehen, dass der Benutzer oder ein Gerät das Medizinprodukt oder die Verpackung des Medizinprodukts fotografiert, vorzugsweise mit einer Kamera, z. B. einer Kamera eines Tischgerätes oder Smartphone-Kamera, oder das Produkt oder die Verpackung mit einem Erfassungsgerät, z. B. einer Kamera, abtastet. Unter Produkt-Informationen sind bevorzugt Informationen zu verstehen, welche sich auf das Medizinprodukt beziehen und vorteilhafterweise die Produkt-ID, ein Verfallsdatum und eine Chargennummer umfassen. Die Produkt-Informationen können darüber hinaus weitere Informationen über das Medizinprodukt enthalten, wie zum Beispiel eine maximale Transport- oder Lagerzeit für einen Impfstoff.

Unter Interpretation der Codedaten ist ferner das Abrufen von Informationen aus den computerlesbaren Codes zu verstehen. Beispielsweise kann ein Code interpretiert werden, um ihn als GS1-Standardcode zu identifizieren, der die Produkt-ID enthält. Die Produkt-ID ist eine Zahl oder eine Kombination aus Zahlen und Buchstaben, die aus dem Code gelesen werden bzw. extrahiert werden kann. Der Code, beispielsweise ein Barcode, umfasst Daten, dies sind beispielsweise die Zahlen, welche durch die Striche codiert sind. Die entsprechende Produkt-Information ergibt sich dann nach Interpretation der Daten, beispielsweise ergibt sich nach Erkennung des Datenformats, dass es sich um eine Produkt-ID handelt.

Die Erfassungsvorrichtung ist bevorzugt konfiguriert, Codes der Standards Global Standards 1 (GS1), Health Industry Bar Code (HIBC), und Pharmazentralnummer (PZN) zu erkennen und die codierten Daten zu extrahieren.

Die Codegenerierungsvorrichtung erzeugt erfindungsgemäß den Resultatcode, indem die Produkt-Daten aller interpretierten Codes sequentiell aneinandergereiht werden. Insbesondere wird hierbei ein gemeinsamer String, also eine Zeichenkette erzeugt, welche alle Produkt-Daten enthält.

Der Resultatcode ist bevorzugt als Barcode ausgebildet. Dadurch ist eine Übermittlung des Resultatcodes an ein KIS mit einer Schnittstelle, welche nur Barcodes akzeptiert, möglich. Bevorzugt werden Datamatrixcodes verwendet. Wenn die Informationen in mehreren Barcodes / Codes stehen, setzt der Multireader Informationen aus mehreren Codes als eine Zeichenkette zusammen. Die Reihenfolge sollte dann 01/17/10 bei LOT bzw. 01/17/21 bei SN sein, wobei die Zahlen 17 und 21 hier für Identifikatoren des entsprechenden Barcodes stehen.

Die Erfassungsvorrichtung ist vorteilhafterweise ausgebildet, Barcodes und/oder Datamatrix-Codes zu erfassen. Die Vorteile von Datamatrix-Codes sind insbesondere, dass diese wenig Platz benötigen und auch gelesen werden können, wenn ca. 30% des Datamatrix-Codes beschädigt sind.

Die Lesevorrichtung umfasst in einer vorteilhaften Ausführungsform eine Übertragungsvorrichtung mit einer Schnittstelle zur Übermittlung des Resultatcodes an ein Krankenhausinformationssystem, wobei die Übertragungsvorrichtung ausgebildet ist, den Resultatcode an das Krankenhausinformationssystem zu übermitteln.

Die Schnittstelle ist dabei vorteilhafterweise als MDM-Schnittstelle nach HL7-Standard ausgebildet
In einer bevorzugten Ausführungsform ist die Lesevorrichtung konfiguriert, auf der Grundlage der aus den Produkt-Daten entnommenen Produkt-Informationen zu bestimmen, ob ein vordefinierter Satz relevanter Informationen für das medizinische Produkt in den Produkt-Informationen des wenigstens einen erfassten Codes und/oder in einer verknüpften Datenbank enthalten ist, welche in einer Speichereinheit der Lesevorrichtung und/oder in einer Speichereinheit eines Servers gespeichert ist.

Unter einem vordefinierten Satz relevanter Informationen ist eine Menge von Informationen zu verstehen, die sich auf das Medizinprodukt beziehen, das gescannt und dokumentiert werden muss. Die vordefinierten relevanten Informationen können z.B. durch gesetzliche Vorgaben, durch den Anwender oder durch unterschiedliche Anforderungen wie z.B. krankenhausinterne Anforderungen, definiert werden.

Die Interpretationsvorrichtung ist bevorzugt dazu ausgebildet, bei einer Übermittlung des Resultatcodes an das Krankenhausinformationssystem die Produkt-Informationen an die Datenbank zu übermitteln. Auf diese Weise kann sichergestellt werden, dass der Datenbankbestand an Daten aktuell gehalten wird.

Die Interpretationsvorrichtung ist vorteilhafterweise konfiguriert, die Produkt-Informationen aus dem wenigstens einen Code mit Produkt-Informationen in der Datenbank abzugleichen und die Produkt-Informationen aus dem wenigstens einen Code in der Datenbank zu speichern, sofern die Produkt-Informationen in der Datenbank nicht abgelegt waren.

Die Datenbank, die beispielsweise in der Cloud eingerichtet ist, kann Anforderungen für die Dokumentationspflicht eines Produkts kennen, beispielsweise: chargenpflichtig (ja / nein), seriennummernpflichtig (ja / nein), Verfallsdatumpflichtig (ja / nein). Sofern ein Produkt bzw. Artikel keine weiteren Daten brauchen, reicht es das Produkt zu identifizieren, dies kann vorzugsweise erfolgen mit Hilfe der EAN 8, EAN 13, GS1-GTIN, PZN. In vielen Fällen sind alle benötigten Informationen in einem maschinenlesbaren Code codiert. Der GS1 sieht hierfür folgende Identifier vor:
(01) - GTIN = Handelsnummer; (10) - Charge; (21) - Seriennummer; (17) - Verfallsdatum.

Die Interpretationsvorrichtung ist vorteilhafterweise konfiguriert, bei der Erfassung einer Mehrzahl von Codes einen Code als relevanten Code auszuwählen, wenn die Produkt-Daten der zumindest eines anderen Codes vollständig in den Code-Daten dieses Codes enthalten ist, und einen zwei oder mehr Codes als relevante Codes auszuwählen, wenn die Produkt-Daten nicht in anderen Codes enthalten sind, und wobei die Codegenerierungsvorrichtung konfiguriert ist, den Resultatcode aus Produkt-Daten des oder der als relevant ausgewählten Codes zu bilden. Der/die computerlesbare(n) Code(s) wird/werden somit als relevante(r) Code(s) ausgewählt, wenn er/sie die vordefinierte Menge an relevanten Informationen enthält/enthalten. Bei dem relevanten / den relevanten Code8s) kann es sich um einen einzigen Code handeln, der alle relevanten Informationen enthält, oder um eine Reihe von Codes, welche die relevanten Informationen kombiniert enthalten.

In einer bevorzugten Ausführungsform umfasst die Lesevorrichtung eine Anzeigevorrichtung, welche derart konfiguriert ist, dass sie ein Überlagerungsbild mit dem wenigstens erfassten Codes Bild und visuellen Informationen darstellt. Die Anzeigevorrichtung ist vorteilhafterweise als Touch-Display ausgebildet bzw. umfasst ein Touch-Display. Dies ermöglicht auch die Nutzung von Bereichen des Touch-Displays als Eingabevorrichtung zur Eingabe von Daten durch den Benutzer.

Die visuellen Informationen umfassen vorzugweise wenigstens eine farbige Markierung. Auf diese Weise sind die visuellen Informationen für den Benutzer direkt sichtbar. Die farbige Markierung wird vorzugsweise wenigstens teilweise transparent auf oder überlappend mit dem jeweiligen erfassten Code dargestellt, um ihre Zuordnung zum jeweiligen Code durch den Benutzer zu erleichtern.

Die Farbe der wenigstens einen farbigen Markierung ist vorteilhafterweise abhängig von der Art und/oder Menge der Produkt-Informationen.

Die Anzeigevorrichtung umfasst in einer bevorzugten Ausführungsform ein Anzeigefeld für Instruktionen für den Benutzer.

In einer bevorzugten Ausführungsform umfasst die Lesevorrichtung eine Eingabevorrichtung zur Eingabe von Informationen durch den Benutzer. Über die Eingabevorrichtung kann der Benutzer beispielsweise Vorgänge bestätigen oder abbrechen oder manuelle Dateneingaben durchführen.

Wenn das Produkt bekannt/unbekannt ist und die Informationen vollständig sind, erfolgt ein bevorzugt ein direktes Senden an das KIS ohne vorherige Bestätigung. Wenn das Produkt bekannt ist und die Informationen unvollständig, werden vorteilhafterweise entweder weitere Codes erkannt (es erfolgt dann vollständiges Senden ohne Bestätigen), oder der Anwender bestätigt und ergänzt fehlende Daten z.B. auf dem Touchscreen. Wenn das Produkt unbekannt und die Informationen unvollständig sind, benötigt es Interpretation des Anwenders bzw. Benutzers, welche Informationen für dieses Produkt zu dokumentieren sind, insbesondere
∘ Charge (ja / nein)
∘ Seriennummer (ja / nein)
∘ Verfallsdatum (ja / nein).

Die Produkt-Informationen umfassen bevorzugt die Produkt-ID und/oder ein Verfallsdatum und/oder eine Chargennummer.

In einer bevorzugten Ausführung ist die Lesevorrichtung als Tischgerät zum stationären Einsatz ausgebildet.

Bevorzugt umfasst die Lesevorrichtung eine Beleuchtungseinheit zur Beleuchtung der zu erfassenden Codes.

In einer alternativen bevorzugten Ausführung ist die Lesevorrichtung als mobiles Endgerät, insbesondere Smartphone oder Tablet, ausgebildet.

Die Erfassungsvorrichtung, die Interpretationsvorrichtung, die Anzeigevorrichtung und die Codegenerierungsvorrichtung können hardware- und/oder softwareseitig in der Lesevorrichtung implementiert sein.

Zusammenfassend umfasst die Lesevorrichtung erfindungsgemäß eine Mehrzahl von Vorrichtungen, deren jeweilige Funktion und Zusammenwirken mit den anderen Vorrichtung kurz beschrieben wird. Die Erfassungsvorrichtung kann einen oder mehrere computerlesbare Codes wie Barcodes oder 2D-Codes (insbesondere Datamatrix-Codes) scannen. Die Erfassungsvorrichtung identifiziert die computerlesbaren Codes in dem erfassten Bild und überträgt die Produkt-Daten, d.h. die mit Hilfe der Codes codierten Daten, insbesondere Zahlen, der mehreren Codes von der Erfassungsvorrichtung an die Interpretationsvorrichtung, welche die Produkt-Informationen aus den Produkt-Daten abruft. Bei den Produkt-Informationen handelt es sich um Informationen, die sich auf das Medizinprodukt beziehen, z. B. die Produktkennung, die Chargennummer und das Verfallsdatum des Medizinprodukts.

Die Interpretationsvorrichtung ermittelt, ob die vordefinierten relevanten Informationen in den Produktinformationen der relevanten Codes enthalten sind. Die Interpretationsvorrichtung prüft auch bevorzugt, ob fehlende Produktinformationen in der Datenbank gespeichert sind.

Die Interpretationsvorrichtung bzw. Auswertevorrichtung wählt einen Code als relevanten Code aus, wenn alle Produktinformationen der anderen Codes ebenfalls in diesem einen Code enthalten sind. Wenn es nicht einen Code gibt, der alle Produktinformationen der anderen Codes enthält, können mehrere Codes die relevanten Codes sein. Ein relevanter Code ist jeder Code, der neue Produktinformationen enthält. Wenn also mehrere Codes relevant sind, kombinieren die in den Codes enthaltenen Informationen den vordefinierten Satz an relevanten Informationen.

Die abgerufenen Produkt-Informationen werden bevorzugt in der (Stammdaten-) Datenbank durch die Lesevorrichtung gespeichert. Die Auswerteeinrichtung überprüft bevorzugt, ob die Produktinformation des Erfassten die vordefinierten relevanten Informationen enthält, oder ob die vordefinierten relevanten Informationen stattdessen aus der Datenbank abgerufen werden können. Es ist möglich, die Produktinformation aus den Codedaten und die aus der Datenbank abgefragte Produktinformation zu kombinieren, um die vordefinierte relevante Information zu erhalten. Die Codegenerierungsvorrichtung erzeugt einen Code, welcher zur Übermittlung an das KIS geeignet ist.

In Bezug auf das Lesesystem wird die oben genannte Aufgabe erfindungsgemäß gelöst durch ein Lesesystem mit den Merkmalen von Anspruch 10. Das Lesesystem zum Erfassen mehrerer computerlesbarer Codes eines Medizinprodukts oder einer Verpackung eines Medizinprodukts zur Unterstützung eines Anwenders umfasst dabei mindestens eine oben beschriebene Lesevorrichtung als Client-Gerät, weiterhin umfassend einen zentralen Server, der über einen Speicher mit einer Datenbank verfügt, wobei der Server mit der mindestens einen Lesevorrichtung verbunden ist, um Daten zu und von der Lesevorrichtung zu übertragen.

Die Schnittstelle der Übertragungsvorrichtung und/oder die Schnittstelle zum Server können jeweils kabelgebunden oder drahtlos ausgebildet sein. Bei einer drahtlosen Schnittstelle werden die Daten drahtlos an das KIS gesendet. Es müssen nicht zwingend die Daten live gesendet werden. Die Daten könnten im mobilen Gerät gesammelt werden und dann in einer Verarbeitungsprozedur sukzessive an das KIS gesendet werden.

In Bezug auf das Verfahren wird die oben genannte Aufgabe erfindungsgemäß gelöst mit den Merkmalen von Anspruch 11. Das Verfahren umfasst einen ersten Schritt, nämlich die Aufnahme eines Bildes des Medizinprodukts oder seiner Verpackung mit den Codes durch ein Aufnahmegerät mit einer Kamera. In einem zweiten Schritt erfolgt das Extrahieren mindestens eines Codes in dem aufgenommenen Bild und der darin kodierten Produkt-Daten und das Prüfen, ob vorgegebene Produkt-Informationen in den Produkt-Daten vorhanden sind. In einem dritten Schritt erfolgt das Interpretieren der Produkt-Daten und Abrufen von wenigstens einer Produkt-Information aus den Produkt-Daten, und in einem vierten Schritt wird aus wenigstens einer Produkt-Information eines interpretierten Codes ein Resultatcode erzeugt wird.

Vorteilhafterweise wird der Resultatcode an ein externes System, insbesondere ein Krankenhausinformationssystem, übermittelt.

Der Resultatcode wird erfindungsgemäß erzeugt, indem die Produkt-Daten aller interpretierten Codes, sequentiell aneinandergereiht werden.

Die Erfindung betrifft auch ein computerlesbares Speichermedium mit Befehlen, die, wenn sie von einem Computer ausgeführt werden, den Computer veranlassen, das oben beschriebene Verfahren auszuführen.

Die Vorteile der Erfindung liegen insbesondere darin, dass die Lesevorrichtung mehrere Codes gleichzeitig erfassen kann und aus den darin enthaltenen Daten einen gemeinsamen Resultatcode bzw. zusammenfassenden Code erstellt, welcher an ein externes System, insbesondere in KIS, übermittelt wird.

Aus einer Menge von mehreren computerlesbaren Codes wird der beste Code für den Benutzer hervorgehoben und es werden zusätzliche Informationen bereitgestellt. Somit weiß der Benutzer genau, welcher Code aus einer Reihe von Codes der beste verfügbare Code ist. Wenn es nicht einen einzigen Code auf dem Medizinprodukt oder der Verpackung gibt, der alle relevanten Informationen enthält, können die Produktinformationen aus mehreren Codes kombiniert werden, um die relevanten Informationen zu sammeln.

Dadurch, dass der Benutzer eine Rückmeldung, ob der einzelne Code alle relevanten Informationen enthält oder nicht, kann die Nachbestellung von Medizinprodukten zur Auffüllung des Lagerbestands auf eine vorgegebene Anzahl von Artikeln leicht automatisiert werden, wenn sichergestellt ist, dass das Informationssystem in Echtzeit über korrekte Nutzungsinformationen verfügt.

Ein Ausführungsbeispiel der Erfindung wird anhand einer Zeichnung näher erläutert. Darin zeigen in stark schematisierter Darstellung:
- FIG. 1: eine Lesevorrichtung in einer bevorzugten Ausführungsform;
- FIG. 2: eine Anzeigevorrichtung der Lesevorrichtung gemäß FIG. 1;
- FIG. 3: ein Flussdiagramm eines Verfahrens ein einer bevorzugten Ausführungsform;
- FIG. 4: einen beispielhaften zweidimensionalen Datamatrix-Code;
- FIG. 5: zwei beispielhafte Barcodes;
- FIG. 6: zwei beispielhafte Barcodes mit Zeichenketten, und
- FIG. 7: einen beispielhaften Ausschnitt einer Verpackung mit einem Datamatrix-Code und einem Barcode.

Gleiche Teile sind in allen Figuren mit denselben Bezugszeichen versehen.

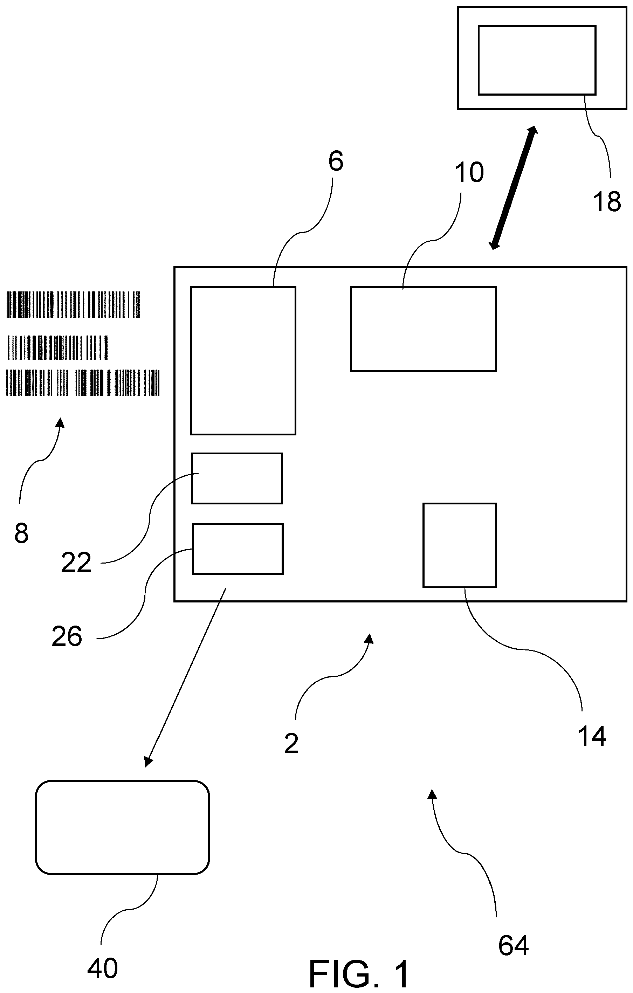

Die FIG. 1 zeigt eine schematische Ansicht einer Lesevorrichtung 2 gemäß einer bevorzugten Ausführungsform. Die Lesevorrichtung 2 umfasst eine Erfassungseinrichtung 6, eine Interpretationsvorrichtung 10, eine Anzeigevorrichtung 14, eine Datenbank 18, eine Codegenerierungsvorrichtung 22 und eine Übertragungsvorrichtung 26.

Die Erfassungsvorrichtung 6 ist vorzugsweise als Kamera ausgebildet, wobei die Lesevorrichtung 2 in diesem Fall als Smartphone ausgebildet ist. In einer anderen bevorzugten Ausführung ist die Lesevorrichtung 2 als Gerät ausgebildet, welches für einen stationären Einsatz am Dokumentationsarbeitsplatz eines Krankenhausinformationssystems ausgebildet ist und eine Kamera aufweist.

Der Benutzer verwendet die Erfassungsvorrichtung 6, um ein Bild eines Satzes von Barcodes oder anders gearteten Codes 8 zu erfassen oder den Satz von Codes zu scannen. Die Codes 8, die in dem dargestellten Ausführungsbeispiel als Barcodes ausgebildet sind, werden aus dem aufgenommenen Bild extrahiert oder im aufgenommenen Bild erkannt.

Die Interpretationseinrichtung 10 bzw. Auswertevorrichtung dekodiert Codes, woraus sich Produkt-Daten ergeben, und erkennt, welche Produkt-Informationen in den Produkt-Daten der einzelnen Codes 8 gespeichert sind. Die Interpretationseinrichtung 10 stellt dann fest, ob die vordefinierte Menge an relevanten Informationen in den Barcodes oder in einer verknüpften Datenbank 18 vorhanden ist. Die Interpretationsvorrichtung 10 wählt die relevanten Codes aus und hebt die relevanten Codes auf der Anzeigevorrichtung 14 hervor. Die Datenbank 18 kann innerhalb der Lesevorrichtung 2 implementiert sind, oder, wie dargestellt, extern zu ihr angelegt sein.

Die Codegenerierungsvorrichtung 22 erzeugt aus wenigstens einem der von der Interpretationsvorrichtung 10 erkannten Codes, insbesondere aus allen Codes, einen Resultatcode, der vorliegend als Barcode ausgebildet ist und das Format eines Strings, d.h. einer Zeichenkette, hat.

Die Lesevorrichtung 2 umfasst eine Übertragungsvorrichtung 26 bzw. Übermittlungseinheit mit einer Schnittzustelle zu einem Krankenhausinformationssystem 40. Sie überträgt den Resultatcode an das Krankenhausinformationssystem 40.

**In** der FIG. 1 ist auch ein Lesesystem 2 dargestellt, welches die Lesevorrichtung 2 als Client umfasst. Die in der Figur dargestellte Datenbank 18 ist Bestandteil eines zentrale Servers 70, der über einen Speicher mit der Datenbank 18 verfügt, wobei der Server 70 mit der mindestens einen Lesevorrichtung 2 verbunden ist, um Daten zu und von der Lesevorrichtung 2 zu übertragen.

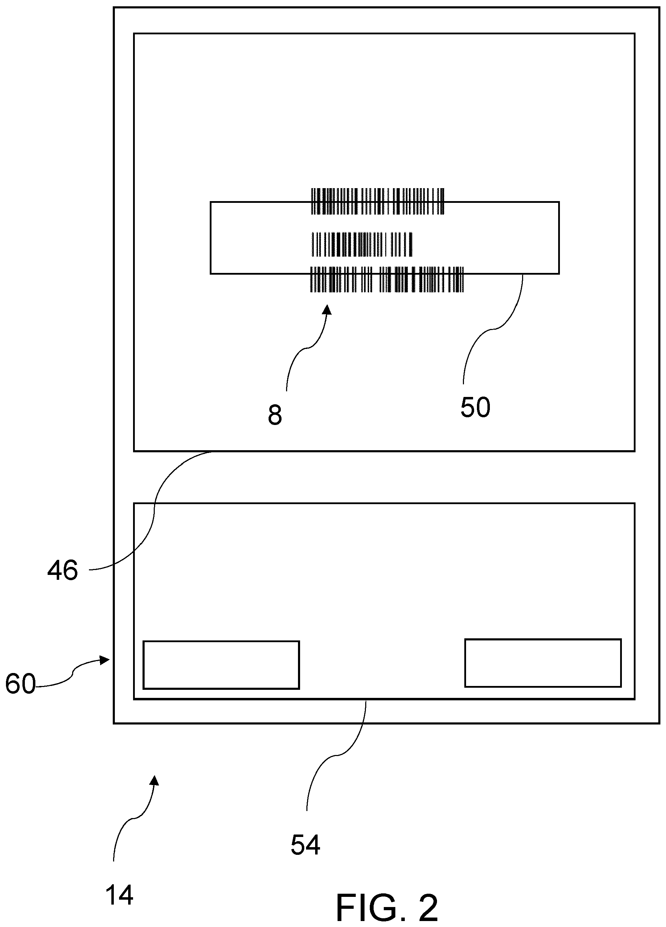

In FIG. 2 ist die Anzeigevorrichtung 14 der Lesevorrichtung 2 gemäß FIG.1 vergrößert dargestellt. Die Anzeigevorrichtung 14 weist einen Überlappungsbereich 46 auf, in dem das Kamerabild des Codes 8 dargestellt, wird, wobei über dem Code 8 ein Marker 50 dargestellt wird. Der Marker 50 wird zumindest teilweise überlappend mit dem Code 8 dargestellt. Dadurch kann der Benutzer den Marker 50 dem Code 8 zuordnen. Eine derartige Zuordnung ist besonders nützlich bei mehreren zeitgleich erfassten Markern. Der Marker wird bevorzugt als zumindest teilweise transparentes, insbesondere rechteckiges, Feld dargestellt, was im Folgenden auch als "Brush" bezeichnet wird.

Die Anzeigevorrichtung 14 weist einen Informationsbereich 54 auf. In diesem Informationsbereich 54 werden die Produkt-Informationen, die von der Interpretationsvorrichtung 10 aus den im Code 8 kodierten Daten entnommen wurden, dargestellt werden. Auf diese Weise erhält der Nutzer unmittelbar eine Rückmeldung über die Informationen, welche aus den Codes extrahiert wurden. Bevorzugt werden die Produkt-Informationen neben Symbolen oder Icons dargestellt, sodass der Benutzer unmittelbar erkennen kann, welche Produkt-Informationen extrahiert werden konnten.

Die Interpretationsvorrichtung 10 ist konfiguriert, die Produkt-Informationen zu validieren und die Anzeigevorrichtung 14 ist konfiguriert, in dem Informationsbereich 54 korrespondierende Nachrichten anzuzeigen. Bevorzugt zeigt die Anzeigevorrichtung im Informationsbereich 54 an, wenn das Verfallsdatum überschritten ist.

Die Anzeigevorrichtung 14 weist eine Eingabevorrichtung 60 auf, die Eingaben von dem Benutzer entgegennimmt. Die Anzeigevorrichtung 14 ist in dem bevorzugten Ausführungsbeispiel als Touch-Display ausgebildet, wobei die Eingabevorrichtung 60 als auf dem Touch-Display eingeblendete Bedienelemente ausgebildet ist.

In dem Fall, dass das Verfallsdatum überschritten ist, werden zwei Bedienelemente eingeblendet mit den Bezeichnungen "Verwenden" und "Nicht verwenden
Bei der Kommunikation mit dem KIS wird die Zeichenkette wird als String an das KIS gesendet. Dies entspricht dem Fall, dass der Anwender einen Barcodescanner / Tastaturbarcodescanner bedienen würde. Die bevorzugte Schnittstelle ist dabei USB.

Ein grüner Marker bzw. "Brush" wird verwendet, wenn der Artikel bekannt ist, insbesondere wenn keine Pflicht zur Dokumentation von Charge, Seriennummer, Verfallsdatum besteht (oder der Artikel ist chargen- / seriennummernpflichtig und die Produktionsinformationen wurden mitverarbeitet). Sofern die vollständigen Informationen aus zwei Codes extrahiert werden, wird auf beiden Codes jeweils ein Marker eingeblendet.

Ein gelber Marker wird verwendet, wenn die erkannten Informationen unvollständig sind. Wenn beispielsweise nur das Produkt erkannt wurde, wird Charge und Verfallsdatum maschinell erfasst oder die fehlenden Informationen werden manuell ergänzt durch Eingaben in der Eingabevorrichtung 60. Wenn z.B. erst einer der beiden Codes erfasst wurde, dann zeigt ein gelber "Brush", dass die Informationen unvollständig ist. Wenn nur Charge / Verfallsdatum gescannt wurden, ist das Produkt unbekannt. Wenn nur das Produkt gescannt wurde, der Artikel aber eine Chargen- oder Seriennummernpflicht hat, ist die Produktion unbekannt. Der Brush ist dabei bevorzugt ein Online-Overlay über den Livestream der Kamera.

Fehlende Informationen wie Verfallsdatum, Charge, Seriennummer können bevorzugt per Handeingabe im Touchscreen eingetippt werden (Tastaturanzeige auf Display).

Ob ein Artikel bekannt wird, überprüft die Lesevorrichtung 2 durch Kommunikation mit der Datenbank 18 (d.h. insbesondere dem KIS in der Cloud).

Für die Zielsetzung des Multireader mit Schnittstelle zum KIS kann man grundsätzlich zwei Ansätze verfolgen:
- Stammdaten müssen im KIS bekannt sein.
- Stammdaten müssen im KIS und in der Cloud bekannt sein.
Die aus den Codes extrahierten Produkt-Daten werden bevorzugt so wie extrahiert (1:1) an das KIS gesendet, unabhängig davon, ob die Produkt-Informationen in der Datenbank 18 hinterlegt sind. Die entsprechenden Produkt-Informationen werden von der Lesevorrichtung 2 in die Datenbank 18 eingespeist.

In einer bevorzugten Ausführungsform wird der Benutzer beim erstmaligen Scannen eines EAN13 / einer GTIN durch eine eingeblendete Nachricht im Informationsbereich 54 gefragt, ob es sich bei dem Produkt, um ein dokumentationspflichtiges Produkt handelt (für welches der Kunde Charge, Seriennummer oder Verfallsdatum dokumentieren möchte). Mit Hilfe entsprechender Eingabefelder in der Eingabevorrichtung 60 kann der Benutzer dies bestätigen und die fehlenden Informationen eingeben, oder die Verpackung enthält weitere Codes.

**In** dem Fall, dass die Lesevorrichtung 2 erkennt, dass wenigstens ein Code fehlt bzw. beschädigt ist und nicht alle notwendigen Informationen aus den Codes extrahiert werden können, wird im Informationsbereich 54 dem Benutzer signalisiert, dass eine manuelle Eingabe der fehlenden Informationen notwendig ist. Der Benutzer kann dies in der Eingabevorrichtung 60 bestätigen. Hierzu kann ein Popup in der Anzeigevorrichtung 14 eingeblendet werden. Zur manuellen Eingabe von fehlenden Informationen wird in der Eingabevorrichtung 60 eine virtuelle Tastatur eingeblendet.

**In** dem Fall, dass Codes von der Erfassungsvorrichtung 6 erfasst werden, welche ein nicht standardisiertes Format aufweisen, wird dem Benutzer in dem Informationsbereich 54 eine Nachricht eingeblendet, dass die Produkt-Informationen manuell erfasst werden müssen. **In** der Eingabevorrichtung 60 wird dazu bedarfsweise eine virtuelle Tastatur eingeblendet. Die Codes werden mit orangefarbenen Markern überblendet.

**In** weiteren Ausführungsformen kann eine mechanische Tastatur zur Eingabe von Produkt-Informationen vorgesehen sein.

Die Lesevorrichtung 2 ist bevorzugt konfiguriert, in der Datenbank 18 enthaltene Produkt-Informationen anzuzeigen und/oder zu ändern und/oder zu löschen.

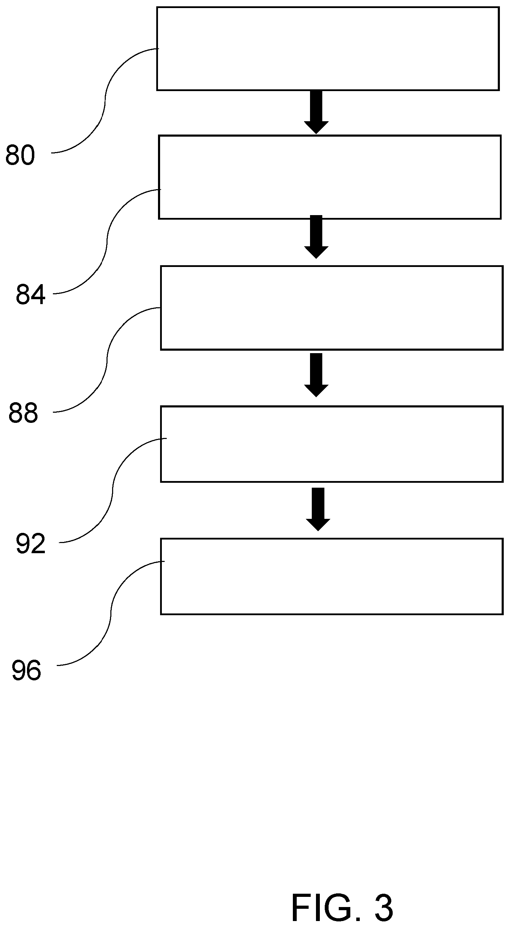

**In** FIG. 3 ist ein Flussdiagramm eines Verfahrens zur Erfassung mehrerer computerlesbarer Codes, vorzugsweise Barcodes und/oder 2D-Codes, eines medizinischen Produkts oder einer Verpackung eines medizinischen Produkts zur Unterstützung eines Benutzers, dargestellt.

In einem ersten Schritt 80 des Verfahrens wird ein Bild eines Medizinprodukts oder seiner Verpackung mit den Codes durch ein Aufnahmegerät mit einer Kamera aufgenommen. Das Aufnahmegerät kann ein Smartphone oder ein Tischgerät mit einer Kamera sein. In einem auf Schritt 80 folgenden zweiten Schritt 84 wird mindestens ein Codes in dem aufgenommenen Bild extrahiert und die in dem Code kodierten Produkt-Daten werden extrahiert.

In einem auf Schritt 84 folgenden dritten Schritt 88 wird wenigstens eine Produkt-Information aus den Produkt-Daten abgerufen. Es wird geprüft, ob vorgegebene Produkt-Informationen in den Produkt-Daten vorhanden sind,
In einem auf Schritt 88 folgenden Schritt 92 wird aus wenigstens einer Produkt-Information eines interpretierten Codes ein Resultatcode erzeugt. In einem such daran anschließenden Schritt 96 wird der Resultat code an ein Krankenhausinformationssystem übermittelt.

Das Verfahren ist software- und/oder hardwaremäßig implementiert, beispielsweise in einem Tischrechner mit einer Kamera oder einem Smartphone.

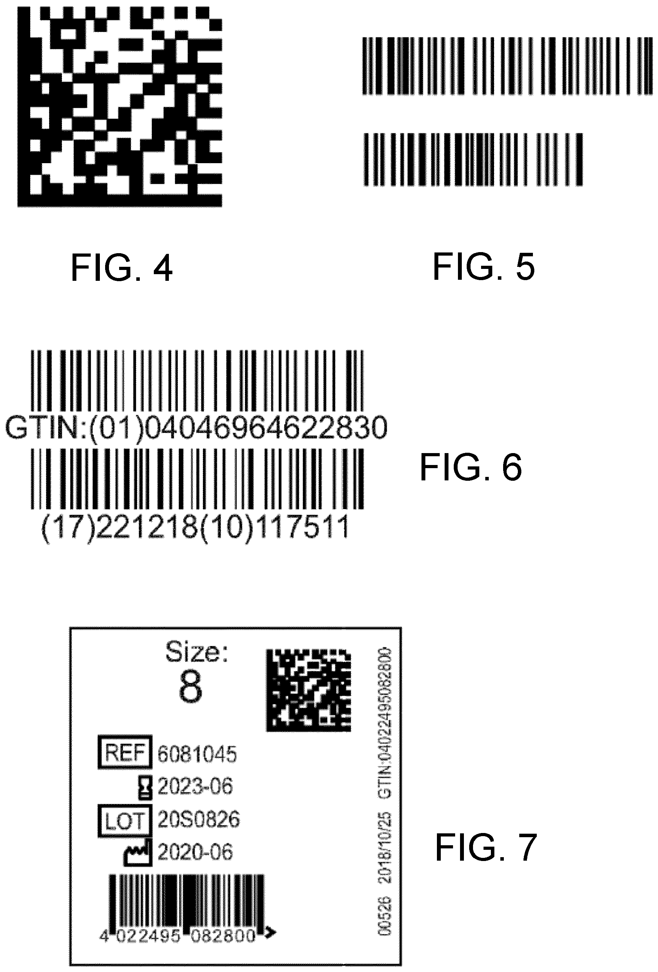

Beispiele für Codes, die von der Lesevorrichtung 2 bzw. mit Hilfe des Verfahrens verarbeitet werden können, zeigen die FIG. 4 bis 7. In FIG. 4 ist ein beispielhafter Datamatrix-Code dargestellt. In der FIG. 5 sind zwei übereinander angeordnete Barcodes dargestellt. In FIG. 6 sind zwei übereinander angeordnete Codes dargestellt, wobei die kodierten Informationen zusätzlich mit Hilfe alphanumerischer Zeichen zusätzlich angegeben sind. In FIG. 7 ist beispielhaft eine Verpackung eines Medizinproduktes dargestellt, welche einen Barcode und einen Datamatrix-Code enthält. Für weitere Beispiele von Codes und Anordnungen von Codes wird auf die bereits im einleitenden Teil zitierte WO 2022/112606 A1 verwiesen.

### Bezugszeichenliste

- 2: Lesevorrichtung
- 6: Erfassungsvorrichtung
- 8: Codes
- 10: Interpretationsvorrichtung
- 14: Anzeigevorrichtung
- 18: Datenbank
- 22: Codegenerierungsvorrichtung
- 26: Übertragungsvorrichtung
- 40: Krankenhausinformationssystem
- 46: Überlappungsbereich
- 50: Marker
- 54: Informationsbereich
- 60: Eingabevorrichtung
- 64: Lesesystem
- 70: Server
- 80: Schritt
- 84: Schritt
- 88: Schritt
- 92: Schritt
- 96: Schritt

## Patentansprüche

1. Lesevorrichtung (2) zum Erfassen wenigstens eines computerlesbaren Codes (8) eines medizinischen Produkts oder einer Verpackung eines medizinischen Produkts zur Unterstützung eines Benutzers, wobei die Lesevorrichtung (2) umfasst:
• eine Erfassungsvorrichtung (6), die derart konfiguriert ist, dass sie ein Bild des medizinischen Produkts oder seiner Verpackung mit dem wenigstens einen Code erfasst und die in dem Code enthaltenen Produkt-Daten extrahiert,
• eine Interpretationsvorrichtung (10), welche konfiguriert ist, diese Produkt-Daten zu interpretieren und zu prüfen, ob vorgegebene Produkt-Informationen in den Produkt-Daten vorhanden sind,
**dadurch gekennzeichnet, dass**
die Lesevorrichtung (2) eine Codegenerierungsvorrichtung (22) umfasst, welche konfiguriert ist, aus den Produkt-Daten einen Resultatcode zu erzeugen, wobei die Codegenerierungsvorrichtung (22) konfiguriert ist, aus allen interpretierten Produkt-Daten einen gemeinsamen Resultatcode zu erzeugen, und wobei die Codegenerierungsvorrichtung (22) den Resultatcode erzeugt, indem die Produkt-Daten aller interpretierten Codes, sequenziell aneinandergereiht werden.

2. Lesevorrichtung (2) nach Anspruch 1, wobei der Resultatcode als Barcode ausgebildet ist und/oder wobei die Erfassungsvorrichtung (6) ausgebildet ist, Barcodes und/oder Datamatrix-Codes zu erfassen.

3. Lesevorrichtung (2) nach Anspruch 1 oder 2, umfassend eine Übertragungsvorrichtung (26) mit einer Schnittstelle zur Übermittlung des Resultatcodes an ein Krankenhausinformationssystem, wobei die Übertragungsvorrichtung (26) ausgebildet ist, den Resultatcode an das Krankenhausinformationssystem zu übermitteln.

4. Lesevorrichtung (2) nach Anspruch 3, wobei die Interpretationsvorrichtung (10) konfiguriert ist, auf der Grundlage der aus den Produkt-Daten entnommenen Produkt-Informationen zu bestimmen, ob ein vordefinierter Satz relevanter Informationen für das medizinische Produkt in den Produkt-Informationen des wenigstens einen erfassten Codes und/oder in einer verknüpften Datenbank (18) enthalten ist, welche in einer Speichereinheit der Lesevorrichtung (2) und/oder in einer Speichereinheit eines Servers gespeichert ist.

5. Lesevorrichtung (2) nach Anspruch 4, wobei die Interpretationsvorrichtung (10) ausgebildet ist, bei einer Übermittlung des Resultatcodes an das Krankenhausinformationssystem die Produkt-Informationen an die Datenbank (18) zu übermitteln und/oder wobei die Interpretationsvorrichtung (10) konfiguriert ist, die Produkt-Informationen aus dem wenigstens einen Code mit Produkt-Informationen in der Datenbank (18) abzugleichen und die Produkt-Informationen aus dem wenigstens einen Code in der Datenbank (18) zu speichern, sofern die Produkt-Informationen in der Datenbank (18) nicht abgelegt waren.

6. Lesevorrichtung (2) nach einem der Ansprüche 1 bis 5, wobei die Interpretationsvorrichtung (10) konfiguriert ist, bei der Erfassung einer Mehrzahl von Codes einen Code als relevanten Code auszuwählen, wenn die Produkt-Daten der zumindest eines anderen Codes vollständig in den Code-Daten dieses Codes enthalten ist, und mehrere Codes als relevante Code auszuwählen, wenn die Produkt-Daten nicht in anderen Codes enthalten sind, und wobei die Codegenerierungsvorrichtung (22) konfiguriert ist, den Resultatcode aus Produkt-Daten des oder der als relevant ausgewählten Codes zu bilden.

7. Lesevorrichtung (2) nach einem der Ansprüche 1 bis 6, umfassend eine Anzeigevorrichtung (14), welche derart konfiguriert ist, dass sie ein Überlagerungsbild mit dem wenigstens erfassten Codes Bild und visuellen Informationen darstellt.

8. Lesevorrichtung (2) nach einem der Ansprüche 1 bis 7, welche als Tischgerät zum stationären Einsatz ausgebildet ist.

9. Lesevorrichtung (2) nach einem der Ansprüche 1 bis 7, welche als mobiles Endgerät, insbesondere Smartphone oder Tablet, ausgebildet ist.

10. Lesesystem (64) zum Erfassen mehrerer computerlesbarer Codes eines Medizinprodukts oder einer Verpackung eines Medizinprodukts zur Unterstützung eines Anwenders, umfassend mindestens eine Lesevorrichtung (2) nach einem der vorherigen Ansprüche als Client-Gerät, weiterhin umfassend einen zentralen Server (70), der über einen Speicher mit einer Datenbank (18) verfügt, wobei der Server (70) mit der mindestens einen Lesevorrichtung (2) verbunden ist, um Daten zu und von der Lesevorrichtung (2) zu übertragen.

11. Computerimplementiertes Verfahren zur Erfassung mehrerer computerlesbarer Codes, vorzugsweise Barcodes und/oder 2D-Codes, eines medizinischen Produkts oder einer Verpackung eines medizinischen Produkts zur Unterstützung eines Benutzers, wobei das Verfahren die folgenden Schritte umfasst:
• Aufnahme eines Bildes des Medizinprodukts oder seiner Verpackung mit den Codes durch ein Aufnahmegerät mit einer Kamera;
• Extrahieren mindestens eines Codes in dem aufgenommenen Bild und der darin kodierten Produkt-Daten;
• Interpretieren der Produkt-Daten und Abrufen von wenigstens einer Produkt-Information aus den Produkt-Daten und Prüfen, ob vorgegebene Produkt-Informationen in den Produkt-Daten vorhanden sind,
**dadurch gekennzeichnet, dass**
aus wenigstens einer Produkt-Information eines interpretierten Codes ein Resultatcode erzeugt wird, und wobei der Resultatcode erzeugt wird, indem die Produkt-Daten aller interpretierten Codes, sequenziell aneinandergereiht werden.

12. Verfahren nach Anspruch 11, wobei der Resultatcode an ein externes System, insbesondere ein Krankenhausinformationssystem, übermittelt wird.

13. Computerlesbares Speichermedium mit Befehlen, die, wenn sie von einem Computer ausgeführt werden, den Computer veranlassen, das Verfahren nach einem der Ansprüche 11 oder 12 auszuführen.

## Claims

1. A reading device (2) for capturing at least one computer-readable code (8) of a medical product or of packaging of a medical product, for supporting a user, wherein the reading device (2) comprises:
• a capturing device (6) which is configured in such a manner as to capture an image of the medical product or of its packaging with the at least one code and to extract the product data contained in the code;
• an interpretation device (10) which is configured to interpret these product data and to check whether predefined product information is present in the product data,
**characterized in that**
the reading device (2) comprises a code generation device (22) which is configured to generate a result code from the product data, wherein the code generation device (22) is configured to generate a common result code from all interpreted product data, and wherein the code generation device (22) generates the result code by sequentially concatenating the product data of all interpreted codes.

2. The reading device (2) according to claim 1, wherein the result code is designed as a barcode and/or wherein the capturing device (6) is designed to capture barcodes and/or data matrix codes.

3. The reading device (2) according to claim 1 or 2, comprising a transmission device (26) having an interface for transmitting the result code to a hospital information system, wherein the transmission device (26) is designed to transmit the result code to the hospital information system.

4. The reading device (2) according to claim 3, wherein the interpretation device (10) is configured, on the basis of the product information taken from the product data, to determine whether a predefined set of relevant information for the medical product is contained in the product information of the at least one captured code and/or in a linked database (18), which is stored in a memory unit of the reading device (2) and/or in a memory unit of a server.

5. The reading device (2) according to claim 4, wherein the interpretation device (10) is designed, upon transmission of the result code to the hospital information system, to transmit the product information to the database (18) and/or wherein the interpretation device (10) is configured to compare the product information from the at least one code with product information in the database (18) and to store the product information from the at least one code in the database (18), provided the product information was not stored in the database (18).

6. The reading device (2) according to any one of claims 1 to 5, wherein the interpretation device (10) is configured to select one code as the relevant code when capturing a plurality of codes, if the product data of the at least one other code are completely contained in the code data of this code, and to select a plurality of codes as relevant codes if the product data are not contained in other codes, and wherein the code generation device (22) is configured to form the result code from product data of the code or codes selected as relevant.

7. The reading device (2) according to any one of claims 1 to 6, comprising a display device (14) which is configured in such a manner as to display a superimposed image with the image of the at least one captured code and visual information.

8. The reading device (2) according to any one of claims 1 to 7, which is designed as a tabletop device for stationary use.

9. The reading device (2) according to any one of claims 1 to 7, which is designed as a mobile terminal device, in particular a smartphone or tablet.

10. A reading system (64) for capturing a plurality of computer-readable codes of a medical product or of packaging of a medical product, for supporting a user, comprising at least one reading device (2) according to any one of the preceding claims as a client device, further comprising a central server (70) which has a memory with a database (18), wherein the server (70) is connected to the at least one reading device (2), in order to transmit data to and from the reading device (2) .

11. A computer-implemented method for capturing a plurality of computer-readable codes, preferably barcodes and/or 2D codes, of a medical product or of packaging of a medical product, for supporting a user, wherein the method comprises the following steps:
• recording an image of the medical product or of its packaging with the codes by a recording device with a camera;
• extracting at least one code in the recorded image and the product data encoded therein;
• interpreting the product data and retrieving at least one product information from the product data and checking whether predefined product information is present in the product data,
**characterized in that**
a result code is generated from at least one piece of product information of an interpreted code, and wherein the result code is generated by sequentially concatenating the product data of all interpreted codes.

12. The method according to claim 11, wherein the result code is transmitted to an external system, in particular a hospital information system.

13. A computer-readable storage medium with instructions which, when executed by a computer, cause the computer to carry out the method according to any one of claims 11 or 12.

## Revendications

1. Dispositif de lecture (2) pour la détection d'au moins un code lisible par ordinateur (8) d'un produit médical ou d'un emballage d'un produit médical, destiné à assister un utilisateur, dans lequel le dispositif de lecture (2) comprend :
• un dispositif de détection (6), qui est configuré pour détecter une image du produit médical ou de son emballage contenant au moins un code et pour extraire les données de produit du produit contenues dans ce code ;
• un dispositif d'interprétation (10), qui est configuré pour interpréter ces données de produit de produit et vérifier la présence d'informations prédéfinies sur le produit dans les données de produit de produit,
**caractérisé en ce que**
le dispositif de lecture (2) comprend un dispositif de génération de code (22), qui est configuré pour générer un code de résultat à partir des données de produit de produit, dans lequel le dispositif de génération de code (22) est configuré pour générer un code de résultat commun à toutes les données de produit interprétées et dans lequel le dispositif de génération de code (22) génère le résultat de code en concaténant séquentiellement les données de produit de tous les codes interprétés.

2. Dispositif de lecture (2) selon la revendication 1, dans lequel le code de résultat est un code-barres et/ou dans lequel le dispositif de détection (6) est configuré pour détecter les codes-barres et/ou les codes Data Matrix.

3. Dispositif de lecture (2) selon la revendication 1 ou 2, comprenant un dispositif de transmission (26) doté d'une interface pour la transmission du code de résultat à un système d'information hospitalier, dans lequel le dispositif de transmission (26) est configuré pour transmettre le code de résultat au système d'information hospitalier.

4. Dispositif de lecture (2) selon la revendication 3, dans lequel le dispositif d'interprétation (10) est configuré pour déterminer, à partir des informations de produit extraites des données de produit, si un ensemble prédéfini d'informations pertinentes pour le produit médical est contenu dans les informations de produit d'au moins un code détecté et/ou dans une base de données de produit liée (18), qui est stockée dans une unité de stockage du dispositif de lecture (2) et/ou dans une unité de stockage d'un serveur.

5. Dispositif de lecture (2) selon la revendication 4, dans lequel le dispositif d'interprétation (10) est configuré pour transmettre les informations de produit à la base de données (18) lors d'une transmission du code de résultat au système d'information hospitalier et/ou dans lequel le dispositif d'interprétation (10) est configuré pour comparer les informations de produit issues d'au moins un code avec les informations de produit présentes dans la base de données (18) et pour stocker les informations de produit issues d'au moins un code dans la base de données (18), à condition que les informations de produit ne soient pas déjà stockées dans la base de données (18).

6. Dispositif de lecture (2) selon une quelconque des revendications 1 à 5, dans lequel le dispositif d'interprétation (10) est configuré pour sélectionner un code comme code pertinent lors de la détection de plusieurs codes détectés, si les données de produit p d'au moins un autre code sont intégralement contenues dans les données de produit de ce code et pour sélectionner plusieurs codes comme codes pertinents, si les données de produit ne sont pas contenues dans d'autres codes, et dans lequel le dispositif de génération de code (22) est configuré pour générer le code de résultat à partir des données de produit du ou des codes sélectionnés comme pertinents.

7. Dispositif de lecture (2) selon une quelconque des revendications 1 à 6, comprenant un dispositif d'affichage (14), qui est configuré pour afficher une image superposée avec au moins l'image du code détecté et des informations visuelles.

8. Dispositif de lecture (2) selon une quelconque des revendications 1 à 7, conçu comme un dispositif de table pour une utilisation stationnaire.

9. Dispositif de lecture (2) selon une quelconque des revendications 1 à 7, configuré comme un terminal mobile, notamment un smartphone ou une tablette.

10. Système de lecture (64) pour la détection de plusieurs codes informatiques d'un produit médical ou d'un emballage d'un produit médical afin d'assister un utilisateur, comprenant au moins un dispositif de lecture (2) selon une quelconque des revendications précédentes en tant que dispositif client, comprenant en outre un serveur central (70) doté d'une mémoire contenant une base de données (18), dans lequel le serveur (70) est connecté à au moins un dispositif de lecture (2) pour le transfert de données vers et depuis le dispositif de lecture (2).

11. Procédé informatique de détection de plusieurs codes lisibles par ordinateur, de préférence des codes-barres et/ou des codes 2D, d'un produit médical ou d'un emballage d'un produit médical, destiné à assister un utilisateur, dans lequel le procédé comprend les étapes suivantes :
• enregistrement d'une image du produit médical ou de son emballage comportant les codes, à l'aide d'un appareil d'enregistrement équipé d'une caméra ;
• extraction d'au moins un code de l'image détectée et des données de produit qui y sont codées ;
• interprétation des données de produit et extraction d'au moins une information de produit à partir des données de produit et vérification de la présence d'informations de produit prédéfinies dans les données de produit,
**caractérisé en ce que**
un code de résultat est généré à partir d'au moins une information de produit d'un code interprété et dans lequel le code de résultat est généré par concaténation séquentielle des données de produit de tous les codes interprétés.

12. Procédé selon la revendication 11, dans lequel le code de résultat est transmis à un système externe, notamment un système d'information hospitalier.

13. Support de mémorisation lisible par ordinateur contenant des instructions qui, lorsqu'elles sont exécutées par un ordinateur, entraînent l'exécution du procédé selon la revendication 11 ou 12.
